# EUROPÄISCHE PATENTSCHRIFT

(11) **EP 3 374 366 B1**
(45) Veröffentlichungstag und Bekanntmachung des Hinweises auf die Patenterteilung: **18.12.2019**
(21) Anmeldenummer: 16745478.4
(22) Anmeldetag: 02.08.2016
(51) Int. Cl.: C07F 15/00, C07B 41/06, C07F 9/145, C07C 29/141, C07C 31/125, C07C 45/50, C07C 47/02, C07C 45/74, C07C 47/21, C07C 29/17

(54) **BIS-PHOSPHITE MIT 2,4-DIMETHYLPHENYL-EINHEITEN UND IHRE VERWENDUNG ALS LIGANDEN IN DER HYDROFORMYLIERUNG**
BIS-PHOSPHITES WITH 2,4-DIMETHYLPHENYL UNITS AND THEIR USE AS LIGANDS IN HYDROFORMYLATION
BISPHOSPHATE PRÉSENTANT DES UNITÉS DE 2,4 DIMÉTHYLPHÉNYL ET SON UTILISATION EN TANT QUE LIGANDS DANS L'HYDROFORMYLATION

(30) Priorität: 09.11.2015 EP 15193607
(43) Veröffentlichungstag der Anmeldung: 19.09.2018
(73) Patentinhaber: Evonik Operations GmbH, 45128 Essen (DE)
(72) Erfinder: DYBALLA, Katrin Marie, 45657 Recklinghausen (DE); FRANKE, Robert, 45772 Marl (DE); FRIDAG, Dirk, 45721 Haltern am See (DE); SCHWARZ, Markus, 45721 Haltern am See (DE); SCHULTE-ALTHOFF, Hermann-Josef, 45721 Haltern am See (DE); GEILEN, Frank, 45721 Haltern am See (DE)
(74) Vertreter: Evonik Patent Association
(86) Internationale Anmeldenummer: PCT/EP2016/068375
(87) Internationale Veröffentlichungsnummer: WO 2017/080690

(56) Entgegenhaltungen:
- WO-A2-2005/009934
- DE-A1-102008 002 187

## Beschreibung

Die Erfindung befasst sich mit der Herstellung von 2-Propylheptanol.

2-Propylheptanol (2PH) ist ein Gemisch aus C₁₀-Alkoholen, welches als Zwischenprodukt für die Herstellung von Weichmachern, Detergenzien und Schmiermitteln verwendet wird.

Die hier besprochene Herstellung von 2PH erfolgt via Rh-katalysierte Hydroformylierung von C₄-Olefin zu C₅-Aldehyd, Aldolkondensation zum C₁₀-Aldehyd und Hydrierung zum C₁₀-Alkohol. Die Syntheseroute an sich ist in EP2280920B1 offenbart. Die vorliegende Anmeldung setzt den Fokus auf die Hydroformylierung und insbesondere auf den der dabei eingesetzten Liganden.

Der in EP2280920B1 verwendete Ligand ist in Formel (7) dargestellt.

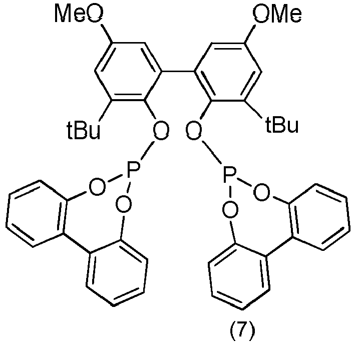

Bei der Hydroformylierung eines Einsatzgemisches, welches 35 % 2-Buten, lediglich 1 % 1-Buten und der Rest ist inertes Butan enthält, wurden mit diesem Katalysatorsystem Butenumsätze von 65 bis 75 % erzielt. Die prozentuale Verteilung zwischen n-Pentanal und 2-Methylbutanal, die so genannte n/iso-Selektivität, betrug mindestens 95 % zu 5 %. Die Regioselektivität für n-Pentanal betrug demnach 95 %.

Die Regioselektivität hinsichtlich eines Zielproduktes ist ein wichtiges Maß für die Effizienz einer Reaktion. Insbesondere, wenn vor allem eines der beiden Produkte bevorzugt gebildet werden soll. Bei der Hydroformylierung gibt es die n/iso-Selektivität das Verhältnis von linearem Aldehyd (= n) zu verzweigtem (= iso) Aldehyd an. Regioselektivität n-Pentanal bedeutet, dass diese Menge an linearem Produkt gebildet wurde. Die restlichen Prozente entsprechen dann dem verzweigten Isomer. Eine hohe Regioselektivität hinsichtlich n-Pentanal bedeutet demnach, dass vergleichsweise viel des begehrten Produktes gebildet wird.

Insoweit ist der in EP2280920B1 verwendete Ligand (7) kaum zu beanstanden. Nachteil des auf Ligand (7) basierenden Katalysatorsystems ist jedoch, dass es nach einer Betriebsdauer von etwa 1000 h einen Niederschlag an der Wand des Reaktors verursacht. Eine Analyse des Niederschlags ergab, dass es sich dabei um phosphorhaltige Folgeprodukte des Bisphosphit-Liganden (7) und des eingesetzten Amins handelt. Dies bedeutet, dass der in EP2280920B1 beschriebene Ligand sich trotz des als Stabilisators eingesetzten Amins bereits nach einer für ein industriell praktizierbares Verfahren relativ kurzen Betriebsdauer abbaut, sodass der Umsatz der Reaktion sinkt.

Signifikante Prozessverbesserungen konnten mit dem in EP2802550B1 beschriebenen Katalysatorsystem erzielt werden, welches mindestens einen Liganden der Formeln (5) und (4) umfasst:

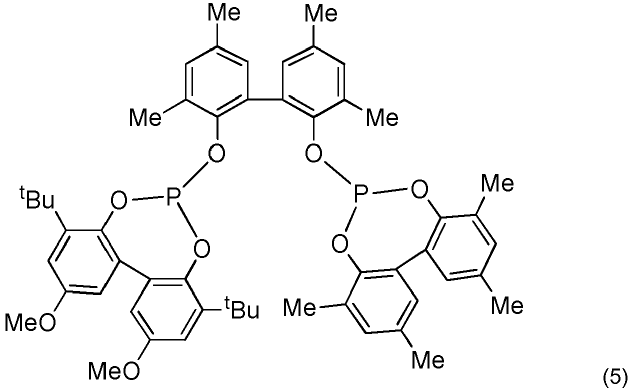

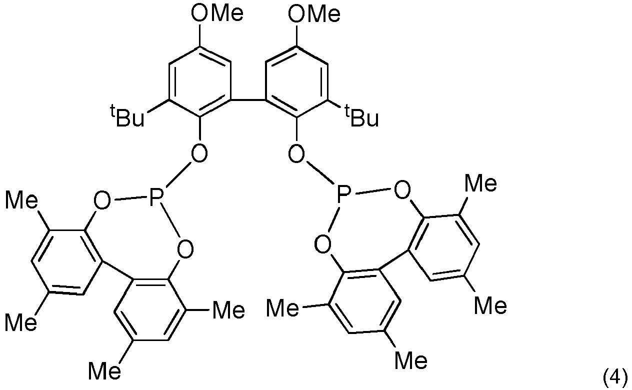

Über 8000 Betriebsstunden wurde kein Feststoffanfall festgestellt. Die Regioselektivität verharrte auf einem hohen Niveau von etwa 93 %; die Ausbeute bewegte sich zwischen 60 % und 80 %.

Nachteil des auf (4) bzw. (5) basierenden Katalysatorsystems gegenüber dem Katalysatorsystem, welches (7) als Ligand einsetzt, ist nicht nur die schlechtere Regioselektivität sondern auch die schlechtere Effizienz der Synthese für die Liganden (4) und (5): Beide Liganden weisen nämlich eine Verbrückung zwischen ihren benachbarten Phenol-Ringen auf, dessen Synthese bei weitem nicht so gute Ausbeuten liefert wie bei dem Biphenolbaustein des Liganden (7). Mithin ist das auf (4) und (5) basierende Katalysatorsystem deutlich aufwendiger und ineffizienter in der Herstellung als dasjenige, welches den Ligand (7) nutzt. Nichtsdestotrotz zeichnet sich ein 2-PH Prozess mit den Liganden (4) und (5) durch die verlängerte Lebensdauer und die bessere Prozessstabilität aus, so dass der in EP2802550B1 beschriebene Prozess im Ergebnis besser ist als der aus EP2280920B1 bekannte.

WO 2005/009934 offenbart ein Verfahren zur Herstellung von 2-Propylheptanol, wobei im Hydroformylierungsschritt Phosphine und Phosphoramidite eingesetzt werden.

Gleichwohl besteht weiterhin Verbesserungsbedarf dahingehend, das Verfahren zur 2PH-Herstellung deutlich effizienter zu machen. Dies ist die Aufgabe, welcher dieser Erfindung zu Grunde liegt.

Gelöst wird diese Aufgabe dadurch, dass in der Hydroformylierung ein Katalysatorsystem eingesetzt wird, das durch weniger Syntheseschritte hergestellt werden kann und welches zugleich eine bessere Regioselektivität erzielt. Dieses Katalysatorsystem enthält Rhodium als Zentralatom und ist mit dem Ligand (1) komplexiert:

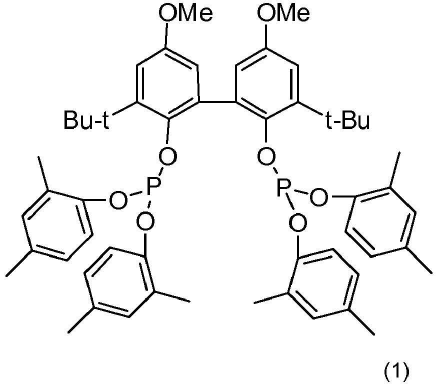

Die IUPAC Bezeichnung von Ligand (1) ist 3,3'-di-tert-butyl-5,5'-dimethoxy-[1,1'-biphenyl]-2,2'-diyltetrakis(2,4-dimethylphenyl)bis(phosphite).

Der Vorteil des Liganden (1) gegenüber (7) ist seine bessere Langzeitstabilität. Gegenüber (5) und (4) ist (1) deutlich effizienter in der Herstellung bzw. der Synthese des Liganden, da der erfindungsgemäß eingesetzte Ligand mit einem Syntheseschritt weniger auskommt. Die Ligandensynthese geht dann schneller, sodass weniger Standzeiten in den Reaktoren für die Ligandenherstellung benötigt werden und es fallen auch weniger Abfallstoffe an. Dies ist insbesondere vor dem Hintergrund der von der chemischen Industrie zunehmend geforderten Nachhaltigkeit ein wichtiges Argument. Darüber hinaus ist die Regioselektivität von (1) deutlich besser als die von (5) und (4), sodass ein damit katalysierter Prozess deutlich mehr begehrtes n-Pentanal bildet als weniger gewünschtes iso-Pentanal. Die Regioselektivität von (1) gegenüber (7) ist geringfügig besser.

Gegenstand der Erfindung ist mithin Verfahren zur Herstellung von 2-Propyl-1-heptanol mit den folgenden Schritten:
a) Bereitstellen eines Einsatzstoffgemisches enthaltend zumindest cis-2-buten und/oder trans-2-buten;
b) Beaufschlagen des Einsatzstoffgemisches mit Kohlenmonoxid und Wasserstoff in Gegenwart eines homogenen Katalysatorsystems, welches Rhodium sowie mindestens eine Organophosphorverbindung als Ligand umfasst, bei einer Temperatur zwischen 110°C und 150°C und einem Druck zwischen 10*10⁵ Pa und 30*10⁵ Pa zwecks Durchführung einer Hydroformylierung unter Erhalt eines ersten Reaktionsgemisches, welches zumindest n-Pentanal und iso-Pentanal enthält;
c) Gewinnen einer Aldehydfraktion enthaltend n-Pentanal und iso-Pentanal aus dem ersten Reaktionsgemisch;
d) Unterwerfen der Aldehydfraktion einer Aldolkondensation in Gegenwart einer wässrigen Base, wobei ein zweites Reaktionsgemisch erhalten wird, welches eine wässrige Phase und eine organische Phase umfasst, wobei die organische Phase zumindest 2-Propylhept-2-enal enthält;
e) Trennen der organischen Phase von der wässrigen Phase;
f) Beaufschlagen der organischen Phase mit Wasserstoff in Gegenwart eines heterogenen Katalysators zwecks Durchführung einer Hydrierung, unter Erhalt eines dritten Reaktionsgemisches, welches zumindest 2-Propyl-1-heptanol enthält;
g) Gewinnen einer Zielfraktion enthaltend 2-Propyl-1-heptanol aus dem dritten Reaktionsgemisch;
wobei die Verbesserung darin besteht,
dass das homogene Katalysatorsystem zumindest die Organophosphorverbindung gemäß Formel (1) als Ligand umfasst:

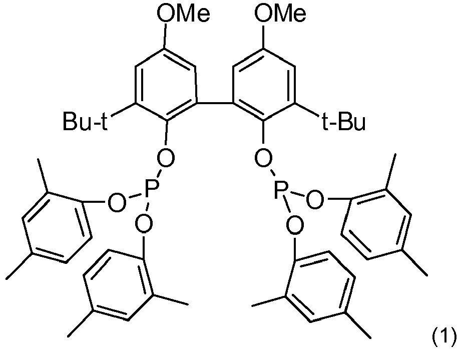

Eine Besonderheit des hier beschriebenen Liganden (1) ist, dass er bei vergleichsweise hohen Temperaturen um 130°C eingesetzt wird. Der Grund dafür ist, dass die Ausbeute dieses Liganden bei 130°C besser ist als bei 120°C. Die Liganden (7) und (4) bzw. (5) werden hingegen ausweislich der Beispiele der EP2280920B1 bzw. der EP2802550B1 bei lediglich 120°C eingesetzt. Mithin sieht eine bevorzugte Weiterbildung der Erfindung es vor, dass Schritt b) des Verfahrens bei einer Temperatur zwischen 120°C und 140°C durchgeführt wird. Der Druck liegt vorzugsweise zwischen 15*10⁵ Pa und 25*10⁵ Pa.

Unter Verwendung vom Ligand (1) bei Temperaturen um 130°C gelingt es ein aus cis-2-Buten und trans-2-Buten gebildetes Substrat zu 50 bis 70 % in der Hydroformylierung umzusetzen (Ausbeute in Richtung der C₅-Aldehyde). Davon entfallen dann 96 % bis 99 % auf n-Pentanal. Die Regioselektivität hinsichtlich n-Pentanal erzielt also einen Wert zwischen 96 % und 99 %. Sofern das Einsatzstoffgemisch auch 1-Buten enthält, gehört dieses im Sinne der Anmeldung ebenfalls zum Substrat und geht in die Berechnung der Regioselektivität mit ein.

Diese hohe Regioselektivität lässt sich - abgesehen von vereinzelten messtechnisch bedingten Ausreißern - über einen Zeitraum mindestens 2000 h einhalten.

Um die Betriebsdauer zu verlängern, wird die Hydroformylierung in Gegenwart eines organischen Amins der Formel (3) durchgeführt wird, 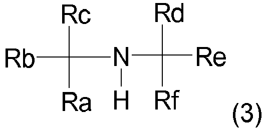 worin Ra, Rb, Rc, Rd, Re und Rf gleiche oder unterschiedliche Kohlenwasserstoffreste darstellen, die auch untereinander verbunden sein können. Das organische Amin weist vorzugsweise zumindest eine 2,2,6,6-Tetramethylpiperidineinheit auf. Konkret kann das organische Amin ein Sebacinsäuredi-4-(2,2,6,6-tetramethylpiperidinyl)-ester sein.

Als Einsatzstoff für die Hydroformylierung hat sich bewährt ein Gemisch welches die folgende, sich zu 100 Gew.-% ergänzende Zusammensetzung aufweist:

| | |
|---|---|
| Summe aus cis-2-Buten und trans-2-Buten: | 10 Gew.-% bis 50 Gew.-%; |
| 1-Buten: | 0 bis 5 Gew.-%; |
| Summe aus n-Butan und Isobutan: | 50 Gew.-% bis 90 Gew.-%; |
| Summe sonstige Stoffe: | 0 bis 1 Gew.-%. |

Das hydroformylierbare Substrat wird demnach im Wesentlichen von dem cis-2-Buten und dem trans-2-Buten gebildet. Sofern in dem Gemisch auch 1-Buten enthalten ist, ist dieses ebenso als Teil des Substrats aufzufassen, da es in der Hydroformylierung ebenfalls n-Pentanal bildet. Ein solches Einsatzstoffgemisch ist vergleichsweise preisgünstig, da es kaum 1-Buten und viel inertes Butan enthält. Da das hier beschriebene Katalysatorsystem so eine gute Regioselektivität aufweist und in der Lage ist, die genannten Substrate mit innenständiger Doppelbindung isomerisierend mit hoher n-Selektivität zu hydroformylieren, kann trotz des hohen Anteils an 2-Buten viel n-Pentanal gebildet werden. Insgesamt wird der Gesamtprozess dadurch besonders effizient.

Es wird empfohlen, eine Rhodiumkonzentration im ersten Reaktionsgemisch zwischen 1 Gew.-ppm und 1000 Gew.-ppm einzustellen. Das Ligand/Rhodium-Verhältnis sollte zwischen 1:1 bis 100:1 betragen, wobei neben der Organophosphorverbindung gemäß Formel (1) kein weiterer Ligand als Teil des homogenen Katalysatorsystems vorzusehen ist. Im industriellen Betrieb ist nicht auszuschließen, dass aufgrund von Verunreinigungen andere Organophosphorverbindungen als 3,3'-di-tert-butyl-5,5'-dimethoxy-[1,1'-biphenyl]-2,2'-diyltetrakis(2,4-dimethylphenyl)bis(phosphite) als Teil des Katalysatorsystems an das Rhodium komplexieren. Derartige Verunreinigungen sind aber bei dem angegebenen Ligand/Rhodium-Verhältnis nicht zu berücksichtigen. Diese Angabe bezieht sich einzig auf Ligand (1) und in beabsichtigter Weise muss kein weiterer Ligand vorgesehen sein.

Abgesehen von den hier beschriebenen besonderen Vorgaben wird die Hydroformylierung wie im Stand der Technik üblich betrieben. Die übrigen Syntheseschritte erfolgen ebenfalls in herkömmlicher Art und Weise. Beispielhaft wird auf folgende Druckschriften verwiesen: Hydroformylierung: EP2280920B1 (Aufarbeitung der Aldehyde), EP2802550B1 (Kreisgas-Verfahren m.W.N.); Aldolkondensatation: DE19957522A1 (Reaktionsbedingungen), DE102009045139A1 (Reaktionstechnik), DE102009001594A1 (Phasentrennung). Die Hydrierung erfolgt ebenfalls nach an sich bekannten Verfahren, beispielsweise im Temperaturbereich von 170°C bis 200 °C bei einem Druck von 15*10⁵ Pa bis 30*10⁵ Pa an einem Trägerkatalysator, der als aktive Komponenten zumindest Nickel und Kupfer enthält; vgl. EP3037400A1.

Die Erfindung soll nun anhand von Beispielen näher erläutert werden.

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR31P = SR_{1H} * (BF_{31P} / BF_{1H}) = SR_{1H} * 0.4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Die Synthese der Vergleichsliganden (4) und (5) erfolgt nach dieser Reaktionsgleichung:

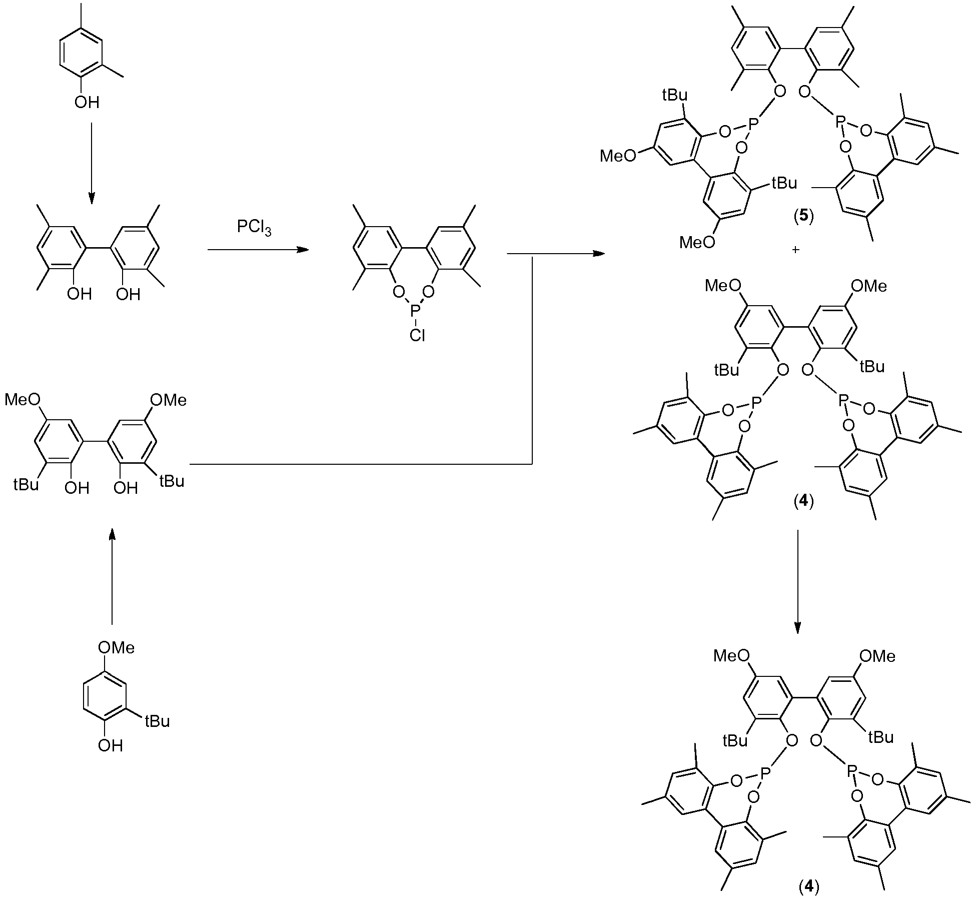

Ein detaillierte Beschreibung der Synthese findet sich in WO 2014/056735A1.

### Die Synthese des erfindungsgemäßen Liganden (1) erfolgt nach dieser Reaktionsgleichung:

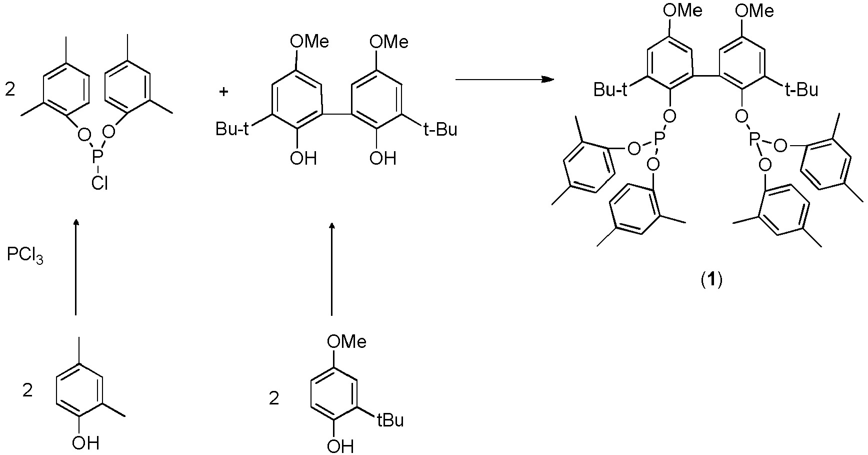

Wie anhand eines Vergleiches der beiden Reaktionsschemata deutlich wird, sind weniger Synthesestufen für die erfindungsgemäße Verbindung (1) nötig als für die in EP2802550B1 verwendeten Verbindungen (4) oder (5). Das 2,4-Dimethylphenol muss bei der Herstellung der Verbindung (1) nicht erst zu einem Biphenol gekuppelt werden, sondern kann direkt mit PCl₃ zu dem entsprechenden Chlorophosphit umgesetzt werden.

### Herstellung von Bis-(2,4-dimethylphenyl)-chlorophosphit:

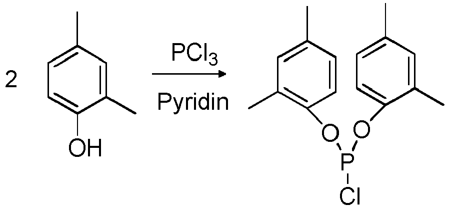

In einem sekurierten 1200 ml Glasreaktor, mit Tropftrichter versehen, wurden 50 g PCl₃ (0.363 mol) und 86 g Pyridin (1.076 mol) in 380 ml getrocknetem Toluol vorgelegt. Die milchig-gelbe PCl₃/Pyridin-Lösung wurde unter Rühren auf -7 °C heruntergekühlt. Dann wurde in den Tropftrichter 86 ml 2,4-Dimethylphenol (0.720 mol) hinzugegeben und in 380 ml getrocknetem Toluol gelöst. Zur Durchführung wurde die Phenol/Toluol-Lösung langsam und stetig zur PCl₃/Pyridin-Lösung hinzugetropft. Über Nacht wurde die Reaktionsmischung unter Rühren auf Raumtemperatur gebracht.

Zur Aufarbeitung wurde das entstandene Hydrochlorid abfiltriert und mit 60 ml getrocknetem Toluol nachgespült und die entstandene Mutterlauge unter vermindertem Druck bis zur Trockne eingeengt. Dabei wird das Lösemittel vollständig entfernt bis ein Feststoff entsteht.

Zur weiteren Aufarbeitung wurde die Rohlösung destilliert. Hierfür wurde ein Spitzkolben mit der Rohlösung befüllt, darauf kam eine kurze Destillationsbrücke ohne Kühlmantel. An der oberen Öffnung wurde das Thermometer platziert, am anderen Ende wurde eine Spinne mit vier weiteren Spitzkolben befestigt. Anschließend wurde diese Apparatur mit einer Kühlfalle verbunden und von dort aus mit der Hochvakuumpumpe. Der Spitzkolben mit dem zu destillierenden Rohliganden wurde mittels Ölbad erwärmt. Zuerst wurde der Vorlauf bei einer Kopftemperatur von 25-30 °C abgenommen. Anschließend wurde die Spinne weiter gedreht und bei einer Kopftemperatur von 140 °C wurde der Hauptlauf abgenommen. Als keine Tropfen mehr im Hauptlauf ankamen, wurde die Destillation abgestellt, die Pumpe runtergefahren und der Hauptlauf in dem entsprechenden Spitzkolben entnommen, verschlossen und analysiert. Im Ergebnis wurde eine Gesamtmasse von 56.7 g erhalten. Dies entspricht einer Ausbeute von 46%.

### Herstellung von 3,3'-di-tert-butyl-5,5'-dimethoxy-[1,1'-biphenyl]-2,2'-diyltetrakis(2,4-dimethylphenyl)bis(phosphite):

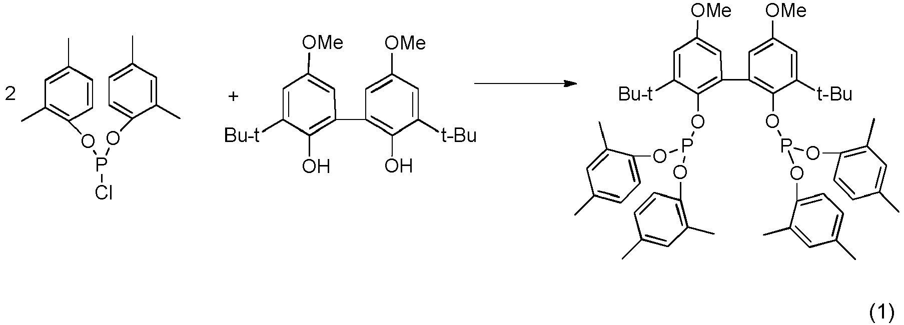

In einem 1000 mL Schlenkkolben wurden zu 51.86 g (0.153 mol) Bis-(2,4-dimethylphenyl)-chlorophosphit unter Rühren bei Raumtemperatur 260 ml getrocknetem Acetonitril zugegeben und das Chlorophosphit aufgelöst.

In einen zweiten 250 ml Schlenkkolben wurden 20.1 g (0.056 mol) 3,3'-Di-tert.-butyl-5,5'-dimethoxy-[1,1'-biphenyl]-2,2'diol mit 12.4 ml (0.153 mol) Pyridin und 155 ml getrocknetem Acetonitril versetzt. Nun wurde die Chlorophosphitlösung im Schlenkkolben auf 0 °C abgekühlt. Anschließend wurde langsam unter kräftigem Rühren die Biphenol/Pyridin-Lösung zugetopft. Die Reaktionsmischung wurde ca. 3 h bei dieser Temperatur gehalten und dann ganz langsam über Nacht auf Raumtemperatur gebracht. Dann wurde die Suspension abfiltriert, mit 30 ml Acetonitril gut nachgewaschen und getrocknet. Im Ergebnis wurde eine Masse von 44.01 g erhalten. Dies entspricht einer Ausbeute von 85 %.

Um in diesem Rohliganden den Chlorgehalt zu senken wurde dieser aufgereinigt. Die angegebenen Chlorgehalte verstehen sich als Gesamtchlorgehalte. Der Gesamtchlorgehalt wird nach Wickbold bestimmt: Probenvorbereitung nach DIN 51408 und Messung per Ionenchromatographie nach DIN EN ISO 10304.

Es wurden 5.15 g 3,3'-di-tert-butyl-5,5'-dimethoxy-[1,1'-biphenyl]-2,2'-diyltetrakis(2,4-dimethylphenyl)bis(phosphite) in einen 250 ml Schlenkkolben mit 15 ml entgastem Toluol und 5 ml Pyridin bei 100 °C zum Rühren gebracht. Nachdem alles gelöst war wurde noch für weitere 15 min die Temperatur gehalten und dann auf 90 °C abgekühlt.

In der Zwischenzeit wurde in einen weiteren 250 ml Schlenkkolben 100 ml Heptan und 5ml Pyridin gegeben und die Lösung wurde auf 0 °C herunter gekühlt. Anschließend wurde die Lösung mit dem 3,3'-di-tert-butyl-5,5'-dimethoxy-[1,1'-biphenyl]-2,2'-diyl tetrakis(2,4-dimethylphenyl)bis(phosphite) über die Fritte in die kalte Heptan/Pyridin Lösung gegeben und für 3 h bei 0 °C gerührt. Auch hierbei fiel nichts aus. Also wurde auch hier mittels Vakuumpumpe das Lösemittel abgezogen bis der Feststoff ausgefallen und getrocknet war. In Ergebnis wurde eine Masse von 3.7 g erhalten. Die Chlorbestimmung ergab einen Wert von 20/20 ppm.

### Mit den so hergestellten Liganden wurden sodann Katalyseversuche durchgeführt. Dabei wurde allgemein wie folgt vorgegangen:

In einem 100 ml-Autoklaven der Fa. Parr Instruments wurde mit Hilfe von Synthesegas (Gemisch aus Wasserstoff und Kohlenmonoxid; volumetrisches Verhältnis CO:H2 = 1:1) reines cis-2-Buten hydroformyliert. Als Precursor wurden Rh(acac)(CO)₂ in Toluol vorgelegt. Der Ligand wurde in molaren Überschüssen von 4:1 relativ zum Rhodium verwendet. Als Stabilisator wurde ein Sebacinsäuredi-4-(2,2,6,6-tetramethylpiperidinyl)-ester (Tinuvin 770DF von BASF) im molaren Verhältnis ca. 1:1 zum Liganden eingesetzt. Zusätzlich wurden als GC-Standard ca. 0.5 g Tetraisopropylbenzol (TIPB) zugefügt. Ca. 6 g Edukt wurden nach Erreichen der vorgesehenen Reaktionstemperatur zudosiert.

Während der Reaktion wurde der Druck über eine Synthesegasregelung mit Massendurchflussmesser konstant gehalten. Die Rührerdrehzahl betrug 1200 min⁻¹. Proben wurden aus der Reaktionsmischung nach 12 Stunden gezogen. Die Ergebnisse der Versuche sind in Tabelle 1 zusammengefasst.

### Hydroformylierung mit erfindungsgemäßem Ligand (1):

In einem 100 ml-Autoklaven der Fa. Parr Instruments wurde bei 120 °C und 20*10⁵ Pa Synthesegasdruck 5.6 g cis-2-Buten hydroformyliert. Als Precursor wurden 0.0056 g Rh(acac)(CO)₂ in 48.8 g Toluol vorgelegt. Als Ligand wurden 0.0779 g Ligand in der Katalysatoransatzlösung eingesetzt. Als organisches Amin wurden 0.0416 g Sebacinsäuredi-4-(2,2,6,6-tetramethylpiperidinyl)-ester (Tinuvin 770DF von BASF) sowie 0.5760 g Tetraisopropylbenzol als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 12 Stunden gezogen.

### Hydroformylierung mit nicht erfindungsgemäßen Liganden (4) und (5):

Zu Vergleichszwecken wurden die Verbindung (5) und sein symmetrisches Isomer Verbindung (4) unter denselben Bedingungen getestet. In Tabelle 1 sind die Hydroformylierungsergebnisse von reinem cis-2-Buten bei 20*10⁵ Pa Synthesgasdruck und bei einer Temperatur von 120°C dargestellt.

**Tabelle 1: Ergebnisse der Versuche im Autoklaven**

| Ligand | Ausbeute Aldehyd in [%] | Regioselektivität n-Pentanal in [%] |
|---|---|---|
| (5) | 95 | 94 |
| (4) | 66 | 90 |
| (1) | 95 | 98 |

### Diskussion der Ergebnisse:

Der nicht erfindungsgemäße Ligand (5) weist eine sehr gute n-Pentanal-Regioselektivität von 94% und gute Aldehydausbeuten auf. Sein symmetrisches Isomer (4) weist eine geringere Regioselektivität hinsichtlich n-Pentanal von lediglich 90% und deutlich geringere Ausbeuten auf. Die beste Regioselektivität wird von dem erfindungsgemäßen Liganden (1) mit 98 % erzielt. Diese liegt sogar noch höher als die 95 %, die der nicht erfindungsgemäße Ligand (7) in EP2280920B1 erreichte.

### Auch im Langzeitversuch konnte Ligand (1) überzeugen:

### Beispiel 1: Hydroformylierung mit dem nicht erfindungsgemäßen Liganden (7)

Der aus EP2280920B1 bekannte, nicht erfindungsgemäße Ligand der Formel (7) wurde in der Hydroformylierung einer Buten/Butan-Mischung eingesetzt. Dabei wurde Ligand (7) mit Sebacinsäuredi-4-(2,2,6,6-tetramethylpiperidinyl)-ester stabilisiert.

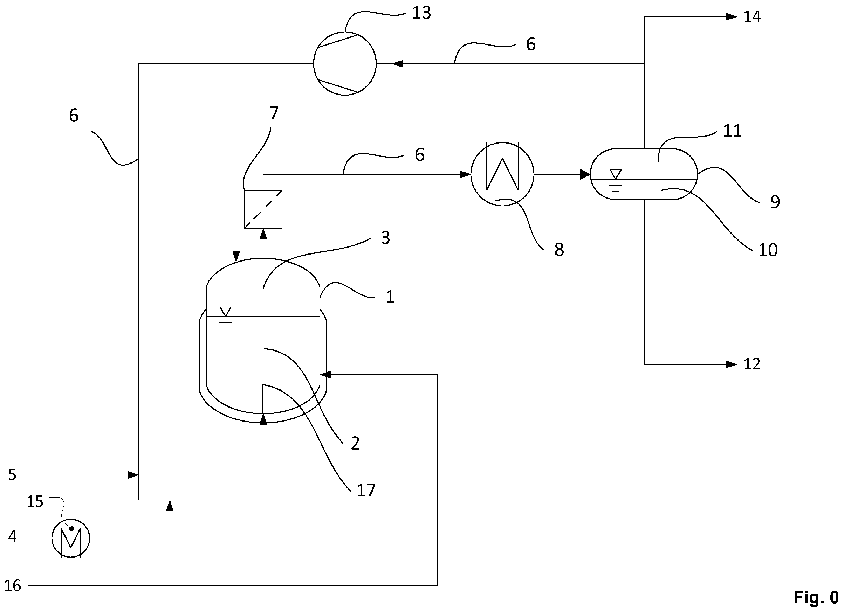

Die kontinuierlich betriebene Versuchsanlage bestand im Wesentlichen aus einem 20 Liter fassenden Druckreaktor mit einem nachgeschalteten Kondensator und Phasentrennbehälter (Gas/Flüssigkeit) für die aus dem Reaktor stammende Gasphase sowie einem Kreisgasverdichter, der die Gasphase aus dem Phasentrennbehälter wieder unten in die Reaktionszone zurück führt. Ein Teil dieses Kreisgases wird nach der Phasentrennung als Abgas aus dem Reaktionssystem gefahren. Um eine optimale Gasverteilung im Reaktorsystem zu realisieren, wurde hier ein Gasverteilerring mit Bohrungen verbaut. Über installierte Heiz- und Kühlvorrichtungen konnte der Reaktor temperiert werden. Die Versuchsanlage ist in Figur 0 schematisch dargestellt.

### Figur 0: Schematische Darstellung der verwendeten Versuchsanlage

Vor der Hydroformylierung wurde das System mit Stickstoff frei von Sauerstoff gespült. Anschließend wurde der Reaktor mit Katalysatorlösung gefüllt. Diese Katalysatorlösung setzte sich zusammen aus 12 kg Vestinol ® INB (CAS 670241-72-2), 4.5 g Rh(acac)(CO)₂, 54.9 g Bisphosphit-Ligand der Formel (7) und 50.4 g Sebacinsäuredi-4-(2,2,6,6-tetramethylpiperidinyl)-ester (Tinuvin 770DF von BASF) und wurde vorher in einem Behälter gemischt. Das Vestinol ® INB wurde zuvor mit Stickstoff gestrippt, um Sauerstoff und Wasser daraus zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 130 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Hierzu wurde ein Einsatzgemisch über einen Verdampfer gefahren, um es gasförmig in das Kreisgas einzuspeisen. Bei dem Einsatzgemisch handelte es sich um eine Mischung aus 35 Gew.-% 2-Buten sowie 1-Buten in einer Konzentration von ca. 1 Gew.-%. Der Rest war n-Butan. Folgende Durchsätze wurden eingestellt: 0.5 kg/h Einsatzgemisch, 350 NI/h Synthesegas (50 Vol.-% H₂ und 50 Vol.-% CO)

Zur Nachdosierung des Bisphosphit-Liganden (7) wurde eine 0.75%-ige Lösung des Bisphosphit-Liganden (7) in n-Pentanal angesetzt, welches zuvor durch Strippen mit Stickstoff von restlichen C₄-Kohlenwasserstoffen (< 3 %) befreit wurde. Das Tinuvin 770DF wurde in einem zweifachen molaren Überschuss zum Bisphosphit-Liganden (7) eingesetzt. Zur besseren Stabilisierung dieser Lösung wurde das Tinuvin 770DF vor dem Bisphosphit-Liganden (7) zur Lösung gegeben.

Die Reaktionsprodukte wurden kontinuierlich über den Kreisgasstrom aus dem Reaktor entfernt und im Kondensator bei 40 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen und mittels Gaschromatographie analysiert. Unter den gewählten Reaktionsbedingungen wurden Aldehydausbeuten von rund 75 % bis 90 % erzielt.

Zur Bestimmung der Konzentration des nicht ans Rhodium gebundenen, freien Liganden wurden Proben aus dem Reaktor entnommen und mittels Flüssigchromatographie (HLPC) untersucht. Durch Dosierung der oben beschriebenen Ligandenlösung wurde die Konzentration des Liganden in der Reaktionslösung im Reaktor auf einen einfachen molaren Überschuss des nichtgebundenen Liganden bezogen auf das eingesetzte Rhodium konstant gehalten.

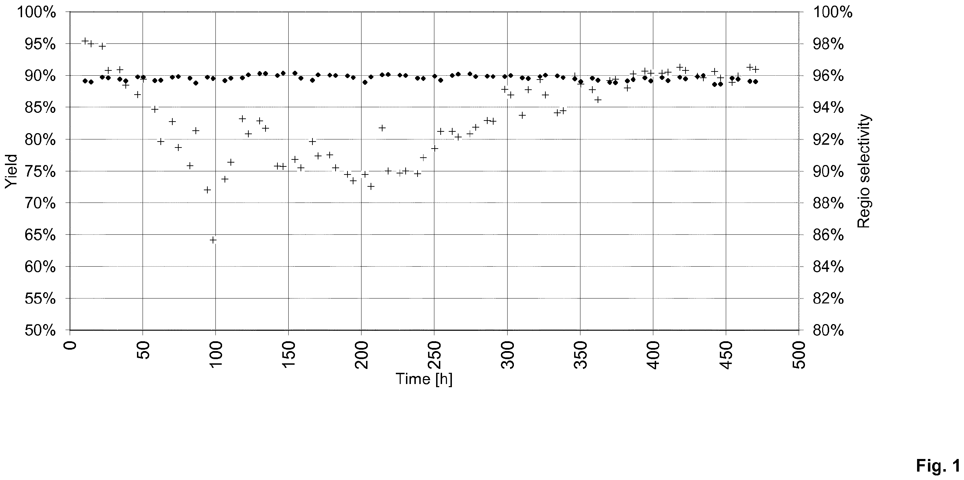

Die prozentuale Verteilung zwischen n-Pentanal und 2-Methylbutanal, bzw. die Regioselektivität, betrug 96 % zu 4 %. Die Ausbeute der C₅-Aldehyde und die Regioselektivität über die Versuchszeit sind in Figur 1 aufgetragen.
- Figur 1:: Diagramm C₅-Ausbeute (Plus +) und Regioselektivität hinsichtlich n-Pentanal (Punkt ●) über die Zeit zu Beispiel 1

### Beispiel 2: Hydroformylierung mit dem erfindungsgemäßen Liganden (1)

Es wurde dieselbe Versuchsanlage eingesetzt wie in Beispiel 1; vgl. Figur 0. Es wurde dasselbe Einsatzgemisch und dasselbe Synthesegas verwendet. Als Ligand wurde indes die Organophosphorverbindung gemäß Formel (1) eingesetzt. Der aus EP2280920B1 bekannte Ligand der Formel (7) war im Reaktionsgemisch nicht enthalten. Es wurde dasselbe Tinuvin 770DF wie im Vergleichsbeispiel 1 als Stabilisator verwendet.

Vor der Hydroformylierung wurde das System mit Stickstoff frei von Sauerstoff gespült. Anschließend wurde der Reaktor mit Katalysatorlösung gefüllt. Diese Katalysatorlösung setzte sich aus 12 kg Vestinol ® INB (CAS 670241-72-2), 4.5 g Rh(acac)(CO)₂, 46.2 g Bisphosphit-Ligand der Formel (1), 49.2 g Tinuvin 770DF zusammen und wurde vorher in einem Behälter gemischt. Das Vestinol ® INB wurde zuvor mit Stickstoff gestrippt, um Sauerstoff und Wasser daraus zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Hierzu wurde ein Einsatzgemisch über einen Verdampfer gefahren, um es gasförmig in das Kreisgas einzuspeisen. Bei dem Einsatzgemisch handelte es sich wieder um die Mischung aus 35 Gew.-% 2-Buten und 1-Buten in einer Konzentration von ca. 1 Gew.-%. Der Rest war n-Butan. Folgende Durchsätze wurden eingestellt: 0.5 kg/h Einsatzgemisch, 270 NI/h Synthesegas (50 Vol.-% H₂ und 50 Vol.-% CO)

Zur Nachdosierung des Bisphosphit-Liganden (1) wurde eine 0.75%-ige Lösung des Bisphosphit-Liganden (1) in n-Pentanal angesetzt, welches zuvor durch Strippen mit Stickstoff von restlichen C₄-Kohlenwasserstoffen (< 3 %) befreit wurde. Das Tinuvin 770DF wurde in einem zweifachen molaren Überschuss zum Bisphosphit-Liganden (1) eingesetzt. Zur besseren Stabilisierung dieser Lösung wurde das Tinuvin 770DF vor dem Bisphosphit-Liganden (1) zur Lösung gegeben.

Die Reaktionsprodukte wurden kontinuierlich über den Kreisgasstrom aus dem Reaktor entfernt und im Kondensator bei 40 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen und mittels Gaschromatographie analysiert.

Unter den gewählten Reaktionsbedingungen wurden Aldehydausbeuten von rund 40 % bis 50 % erzielt.

Zur Bestimmung der Konzentration, des nicht ans Rhodium gebundenen Liganden, wurden Proben aus dem Reaktor entnommen und mittels Flüssigchromatographie (HLPC) untersucht. Durch Dosierung der oben beschriebenen Ligandenlösung wurde die Konzentration des Liganden in der Reaktionslösung im Reaktor auf einen einfachen molaren Überschuss des nichtgebundenen, freien Liganden bezogen auf das eingesetzte Rhodium konstant gehalten.

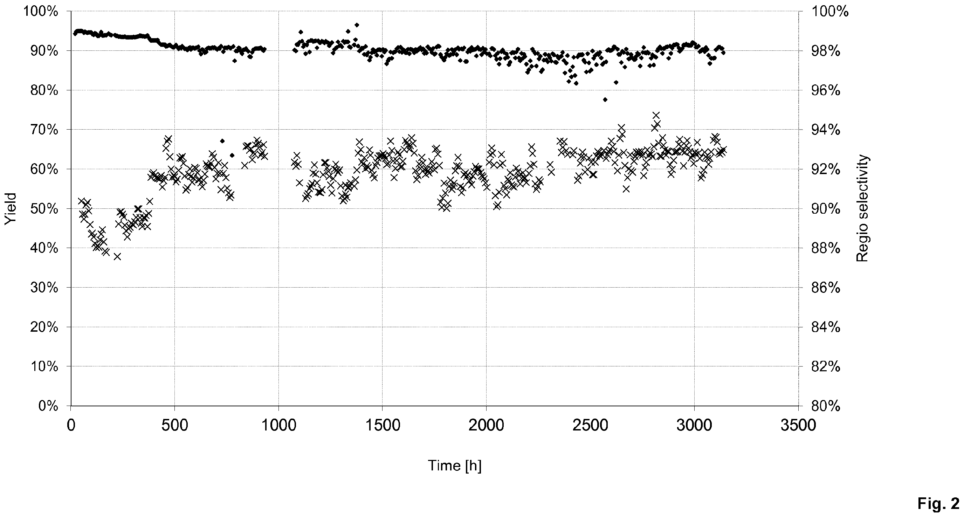

Nach 350h wurde die Reaktionstemperatur von 120°C auf 130°C angehoben. Dies führte zu einer Ausbeutesteigerung und die Ausbeute erreichte Werte zwischen 50 % und 70 %. Die prozentuale Verteilung zwischen n-Pentanal und 2-Methylbutanal, also die Regio-Selektivität, betrug mindestens 97 % n-Pentanal zu 3 % 2-Methylbutanal. Die Ausbeute der C₅-Aldehyde und die Regioselektivität über die Versuchszeit sind in Figur 2 aufgetragen.
- Figur 2:: Diagramm C₅-Ausbeute (Kreuz **x**) und Regio-Selektivität hinsichtlich n-Pentanal (Raute ◆) über die Zeit zu Beispiel 2

Über den Versuchszeitraum wurden keine Niederschläge in der Versuchsanlage beobachtet.

### Beispiel 3: Hydroformylierung mit dem nicht erfindungsgemäßen Liganden (5)

Der aus EP2802550B1bekannte, nicht erfindungsgemäße Ligand der Formel (5) wurde in der Hydroformylierung einer Buten/Butan-Mischung eingesetzt. Dabei wurde Ligand (5) mit Sebacinsäuredi-4-(2,2,6,6-tetramethylpiperidinyl)-ester stabilisiert.

Es wurde abermals die in Figur 0 dargestellte auch in den Beispielen 1 und 2 verwendete Versuchsanlage eingesetzt.

Vor der Hydroformylierung wurde das System mit Stickstoff frei von Sauerstoff gespült. Anschließend wurde der Reaktor mit Katalysatorlösung gefüllt.

Diese Katalysatorlösung setzte sich aus 12 kg Vestinol ® INB (CAS 670241-72-2) 4.5 g Rh(acac)(CO)₂, 47.1 g Bisphosphit-Ligand der Formel (5), 50.4 g Tinuvin 770DF zusammen und wurde vorher in einem Behälter gemischt. Das Vestinol ® INB wurde zuvor mit Stickstoff gestrippt, um Sauerstoff und Wasser daraus zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 130 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich wieder um die Mischung aus 35 Gew.-% 2-Buten und 1-Buten in einer Konzentration von ca. 1 Gew.-%. Der Rest war n-Butan. Folgende Durchsätze wurden eingestellt: 0.5 kg/h Einsatzgemisch, 250 NI/h Synthesegas (50 Vol% H₂ und 50 Vol% CO).

Zur Nachdosierung des Bisphosphit-Liganden (5) wurde eine 0.75%-ige Lösung des Bisphosphit-Liganden (5) in n-Pentanal angesetzt, welches zuvor durch Strippen mit Stickstoff von restlichen C₄-Kohlenwasserstoffen (< 3 %) befreit wurde. Das Tinuvin 770DF wurde in einem zweifachen molaren Überschuss zum Bisphosphit-Liganden (5) eingesetzt. Zur besseren Stabilisierung dieser Lösung wurde das Tinuvin 770DF vor dem Bisphosphit-Liganden (5) zur Lösung gegeben.

Die Reaktionsprodukte wurden kontinuierlich über den Kreisgasstrom aus dem Reaktor entfernt und im Kondensator bei 40 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen und mittels Gaschromatographie analysiert. Unter den gewählten Reaktionsbedingungen wurden Aldehydausbeuten von rund 80 % bis 85 % erzielt.

Zur Bestimmung der Konzentration des nicht ans Rhodium gebundenen, freien Liganden wurden Proben aus dem Reaktor entnommen und mittels Flüssigchromatographie (HLPC) untersucht. Durch Dosierung der oben beschriebenen Ligandenlösung wurde die Konzentration des Liganden in der Reaktionslösung im Reaktor auf einen einfachen molaren Überschuss des nichtgebundenen Liganden bezogen auf das eingesetzte Rhodium konstant gehalten.

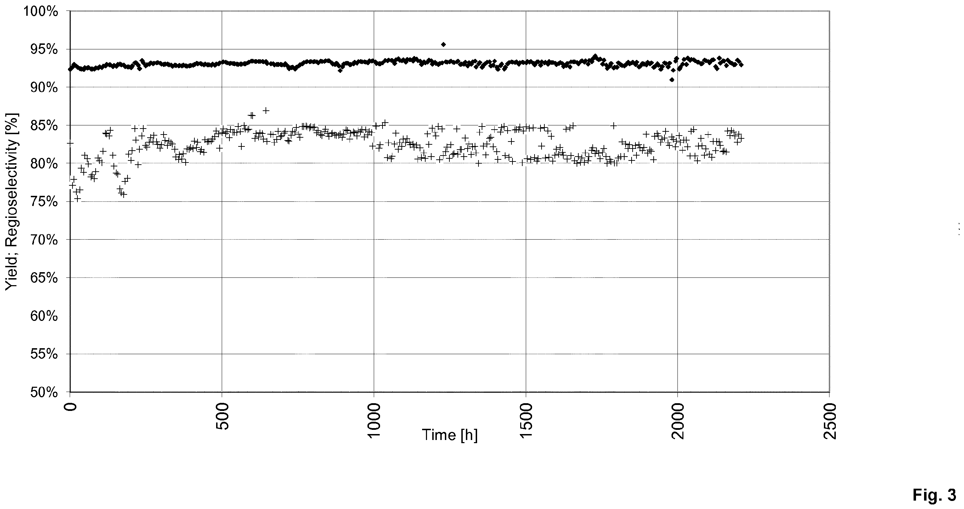

Die prozentuale Verteilung zwischen n-Pentanal und 2-Methylbutanal bzw. die Regioselektivität betrug 93 % zu 7 %. Die Ausbeute der C₅-Aldehyde und die Regioselektivität hinsichtlich n-Pentanal über die Versuchszeit sind in Figur 3 aufgetragen.
- Figur 3:: Diagramm C₅-Ausbeute (Plus **+**) und Regio-Selektivität hinsichtlich n-Pentanal (Raute ◆) über die Zeit zu Beispiel 3

### Fazit

Die in den Beispielen 1, 2 und 3 erbrachten Ergebnisse der Langzeitversuche sind in Tabelle 2 zusammengestellt.

**Tabelle 2: Ergebnisse der Langzeitversuche**

| Beispiel Nr. | Formel Ligand | gemäß der Erfindung | Temperatur | C₅-Ausbeute | Regio-selektivität hinsichtlich n-Pentanal |
|---|---|---|---|---|---|
| 1 | (7) | nein | 130°C | 75 % bis 90 % | 96% |
| 2; t< 350h | (1) | ja | 120°C | 40 % bis 50 % | >98% |
| 2; t>350h | (1) | ja | 130°C | 50 % bis 70 % | >97% |
| 3 | (5) | nein | 130°C | 80 % bis 85 % | 93% |

Die durchgeführten Langzeitversuche bestätigen den frühen Laborbefund, dass die erfindungsgemäß eingesetzte Organophosphor-Verbindung (1) eine höhere Regioselektivität hinsichtlich n-Pentanal erzielt als die Liganden (7) und (5) nach dem Stand der Technik. Bei 130°C sind bessere C₅-Ausbeuten zu erwarten als bei 120°C, wenngleich diese hinter denen des Standes der Technik zurück bleiben. Dieser Nachteil wird jedoch durch die bessere Langzeitstabilität als Ligand (7) und die geringere Synthesekosten als Ligand (5) ausgeglichen.

Da die höhere Regioselektivität zu einer erhöhten Produktion des Zwischenproduktes n-Pentanal führt, welches wiederum in der nachfolgenden Aldolkondensation sich schneller zu C₁₀-Aldehyd umsetzt, ist der Gesamtprozess, Herstellung von 2-Propylheptanol aus 2-Buten chemisch deutlich effizienter als die Verfahren nach dem Stand der Technik. Gegenüber dem in EP2280920B1 beschriebenen Verfahren mit Ligand (7) können die Produktionskosten gesenkt werden, da der erfindungsgemäß eingesetzte Ligand (1) längere Standzeiten aufweist. Gegenüber dem in EP2802550B1 beschriebenen Verfahren ist der erfindungsgemäße Prozess kostengünstiger, da der Ligand (7) weniger Syntheseschritte als die Liganden (4) und (5) benötigt und sich deswegen effizienter herstellen lässt. Diese synergetischen Effekte ermöglichen im Ergebnis eine deutlich wirtschaftlichere Herstellung von 2-Propylheptanol.

### Bezugszeichenliste für Figur 0

- 1: Reaktor
- 2: flüssige Phase
- 3: gasförmige Phase
- 4: Einsatzgemisch
- 5: Synthesegas
- 6: Kreisgas
- 7: Aerosolbrecher
- 8: Kondensator
- 9: Phasentrenngefäß
- 10: Kondensat
- 11: nicht kondensierte Anteile des Kreisgases
- 12: Aufarbeitung
- 13: Kreisgasverdichter
- 14: Offgas
- 15: Verdampfer
- 16: Ligandenlösung
- 17: Gasverteiler

## Patentansprüche

1. Verfahren zur Herstellung von 2-Propyl-1-heptanol mit den folgenden Schritten:
a) Bereitstellen eines Einsatzstoffgemisches enthaltend zumindest cis-2-Buten und/oder trans-2-Buten;
b) Beaufschlagen des Einsatzstoffgemisches mit Kohlenmonoxid und Wasserstoff in Gegenwart eines homogenen Katalysatorsystems, welches Rhodium sowie mindestens eine Organophosphorverbindung als Ligand umfasst, bei einer Temperatur zwischen 110°C und 150°C und einem Druck zwischen 10*10⁵ Pa und 30*10⁵ Pa zwecks Durchführung einer Hydroformylierung unter Erhalt eines ersten Reaktionsgemisches, welches zumindest n-Pentanal und iso-Pentanal enthält;
c) Gewinnen einer Aldehydfraktion enthaltend n-Pentanal und iso-Pentanal aus dem ersten Reaktionsgemisch;
d) Unterwerfen der Aldehydfraktion einer Aldolkondensation in Gegenwart einer wässrigen Base, wobei ein zweites Reaktionsgemisch erhalten wird, welches eine wässrige Phase und eine organische Phase umfasst, wobei die organische Phase zumindest 2-Propylhept-2-enal enthält;
e) Trennen der organischen Phase von der wässrigen Phase;
f) Beaufschlagen der organischen Phase mit Wasserstoff in Gegenwart eines heterogenen Katalysators zwecks Durchführung einer Hydrierung, unter Erhalt eines dritten Reaktionsgemisches, welches zumindest 2-Propyl-1-heptanol enthält;
g) Gewinnen einer Zielfraktion enthaltend 2-Propyl-1-heptanol aus dem dritten Reaktionsgemisch;
wobei die Verbesserung darin besteht,
dass das homogene Katalysatorsystem zumindest die Organophosphorverbindung gemäß Formel (1) als Ligand umfasst:

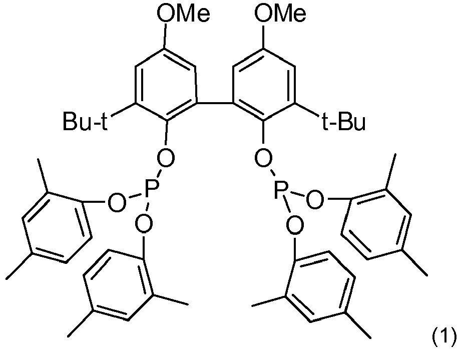

2. Verfahren nach Anspruch 1, **dadurch gekennzeichnet, dass** die Hydroformylierung in Schritt b) bei einer Temperatur zwischen 120°C und 140°C und bei einem Druck zwischen 15*10⁵ Pa und 25*10⁵ Pa durchgeführt wird.

3. Verfahren nach Anspruch 1 oder 2, wobei das im Einsatzstoffgemisches enthaltende cis-2-Buten und trans-2-Buten sowie etwaig im Einsatzstoffgemisch ebenfalls enthaltendes 1-Buten zusammen ein Substrat bilden, **dadurch gekennzeichnet, dass** 96 % bis 99 % des umgesetzten Substrats in n-Pentanal umgesetzt wird (Regioselektivität hinsichtlich n-Pentanal).

4. Verfahren nach Anspruch 3, durchgeführt über einen Zeitraum von mindestens 2000 h, **dadurch gekennzeichnet, dass** sich die Regioselektivität hinsichtlich n-Pentanal über den gesamten Zeitraum im Bereich zwischen 96 % und 99% liegt; abgesehen von vereinzelten messtechnisch bedingten Ausreißern.

5. Verfahren nach Anspruch 1 oder einem der Ansprüche 2 bis 4, **dadurch gekennzeichnet, dass** die Hydroformylierung in Gegenwart eines organischen Amins der Formel (3) durchgeführt wird, 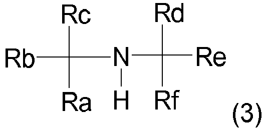 worin Ra, Rb, Rc, Rd, Re und Rf gleiche oder unterschiedliche Kohlenwasserstoffreste darstellen, die auch untereinander verbunden sein können.

6. Verfahren nach Anspruch 5, **dadurch gekennzeichnet, dass** das organische Amin zumindest eine 2,2,6,6-Tetramethylpiperidineinheit aufweist.

7. Verfahren nach Anspruch 6, **dadurch gekennzeichnet, dass** das organische Amin ein Amin ein Sebacinsäuredi-4-(2,2,6,6-tetramethylpiperidinyl)-ester ist.

8. Verfahren nach Anspruch 1 oder einem einem der Ansprüche 2 bis 7, **dadurch gekennzeichnet, dass** das Einsatzstoffgemisch die folgende, sich zu 100 Gew.-% ergänzende Zusammensetzung aufweist:
| | |
|---|---|
| Summe aus cis-2-Buten und trans-2-Buten: | 10 Gew.-% bis 50 Gew.-%; |
| 1-Buten: | 0 bis 5 Gew.-%; |
| Summe aus n-Butan und Isobutan: | 50 Gew.-% bis 90 Gew.-%; |
| Summe sonstige Stoffe: | 0 bis 1 Gew.-%. |

9. Verfahren nach Anspruch 1 oder einem der Ansprüche 2 bis 8, **dadurch gekennzeichnet, dass** die Rhodiumkonzentration im ersten Reaktionsgemisch zwischen 1 Gew.-ppm und 1000 Gew.-ppm beträgt, und dass das Ligand/Rhodium-Verhältnis zwischen 1:1 bis 100:1 beträgt, wobei neben der Organophosphorverbindung gemäß Formel (1) kein weiterer Ligand als Teil des homogenen Katalysatorsystems vorgesehen ist.

## Claims

1. Process for producing 2-propyl-1-heptanol comprising the steps of:
a) providing an input mixture containing at least cis-2-butene and/or trans-2-butene;
b) treating the input mixture with carbon monoxide and hydrogen in the presence of a homogeneous catalyst system comprising rhodium and at least one organophosphorus compound as a ligand at a temperature between 110°C and 150°C and a pressure between 10*10⁵ Pa and 30*10⁵ Pa to perform a hydroformylation to obtain a first reaction mixture containing at least n-pentanal and isopentanal;
c) obtaining an aldehyde fraction containing n-pentanal and isopentanal from the first reaction mixture;
d) subjecting the aldehyde fraction to an aldol condensation in the presence of an aqueous base to obtain a second reaction mixture comprising an aqueous phase and an organic phase, wherein the organic phase contains at least 2-propylhept-2-enal;
e) separating the organic phase from the aqueous phase;
f) treating the organic phase with hydrogen in the presence of a heterogeneous catalyst to perform a hydrogenation to obtain a third reaction mixture containing at least 2-propyl-1-heptanol;
g) obtaining a target fraction containing 2-propyl-1-heptanol from the third reaction mixture;
wherein the improvement consists in the homogeneous catalyst system comprising at least the organophosphorus compound according to formula (1) as a ligand:

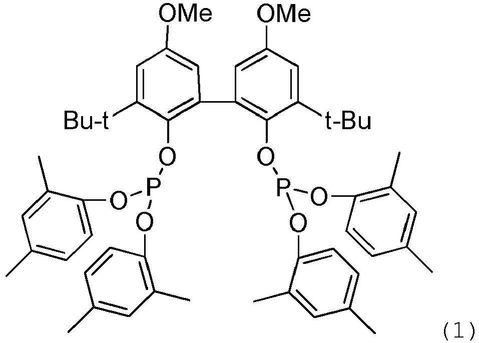

2. Process according to Claim 1, **characterized in that** the hydroformylation in step b) is performed at a temperature between 120°C and 140°C and at a pressure between 15*10⁵ Pa and 25*10⁵ Pa.

3. Process according to Claim 1 or 2, wherein the cis-2-butene and trans-2-butene contained in the input mixture and any 1-butene likewise contained in the input mixture together form a substrate, **characterized in that** 96% to 99% of the converted substrate is converted into n-pentanal (regioselectivity for n-pentanal).

4. Process according to Claim 3, performed over a period of at least 2000 h, **characterized in that** the regioselectivity for n-pentanal is in the range between 96% and 99% over the entire period; except for isolated metrology-dependent outliers.

5. Process according to Claim 1 or any of Claims 2 to 4, **characterized in that** the hydroformylation is carried out in the presence of an organic amine of formula (3) 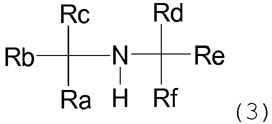 in which Ra, Rb, Rc, Rd, Re and Rf are identical or different hydrocarbyl radicals which may also be joined to one another.

6. Process according to Claim 5, **characterized in that** the organic amine comprises at least one 2,2,6,6-tetramethylpiperidine unit.

7. Process according to Claim 6, **characterized in that** the organic amine an amine is a di-4-(2,2,6,6-tetramethylpiperidinyl) sebacate.

8. Process according to Claim 1 or any of Claims 2 to 7, **characterized in that** the input mixture has the following composition summing to 100% by weight:
| | |
|---|---|
| Sum of cis-2-butene and trans-2-butene: | 10% by weight to 50%byweight; |
| 1-Butene: | 0% to 5%by weight; |
| Sum of n-butane and isobutane: | 50% by weight to 90%byweight; |
| Sum of other substances: | 0 to 1 wt%. |

9. Process according to Claim 1 or any of Claims 2 to 8, **characterized in that** the rhodium concentration in the first reaction mixture is between 1 ppmw and 1000 ppmw and **in that** the ligand/rhodium ratio is between 1:1 to 100:1, wherein no further ligand is provided as part of the homogeneous catalyst system in addition to the organophosphorus compound according to formula (1).

## Revendications

1. Procédé pour la préparation de 2-propyl-1-heptanol comportant les étapes suivantes :
a) mise à disposition d'un mélange de réactants contenant au moins du cis-2-butène et/ou du trans-2-butène ;
b) pressurisation du mélange de réactants avec du monoxyde de carbone et de l'hydrogène en présence d'un système de catalyseur homogène, qui comprend du rhodium ainsi qu'au moins un composé organophosphoré en tant que ligand, à une température entre 110°C et 150°C et à une pression entre 10*10⁵ Pa et 30*10⁵ Pa, pour la réalisation d'une hydroformylation avec obtention d'un premier mélange réactionnel, qui contient au moins du n-pentanal et de l'iso-pentanal ;
c) obtention d'une fraction d'aldéhyde contenant du n-pentanal et de l'iso-pentanal à partir du premier mélange réactionnel ;
d) soumission de la fraction d'aldéhyde à une condensation aldolique en présence d'une base aqueuse, un deuxième mélange réactionnel étant obtenu, qui comprend une phase aqueuse et une phase organique, la phase organique contenant au moins du 2-propylhept-2-énal ;
e) séparation de la phase organique de la phase aqueuse ;
f) pressurisation de la phase organique avec de l'hydrogène en présence d'un catalyseur hétérogène pour la réalisation d'une hydrogénation, avec obtention d'un troisième mélange réactionnel, qui contient au moins du 2-propyl-1-heptanol ;
g) obtention d'une fraction cible contenant du 2-propyl-1-heptanol à partir du troisième mélange réactionnel ;
l'amélioration consistant en le fait que le système de catalyseur homogène comprend au moins le composé organophosphoré selon la formule (1) en tant que ligand :

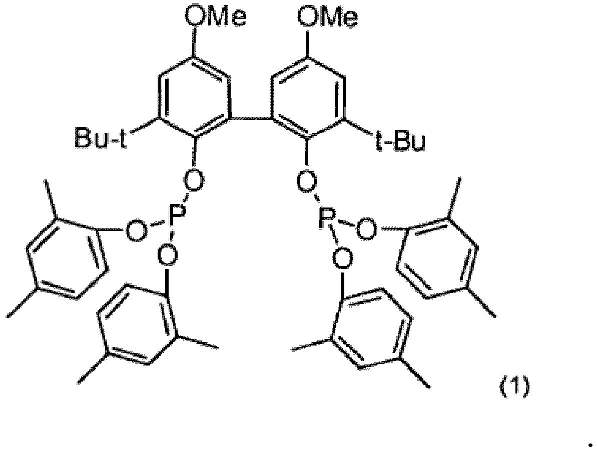

2. Procédé selon la revendication 1, **caractérisé en ce que** l'hydroformylation dans l'étape b) est réalisée à une température entre 120°C et 140°C et à une pression entre 15*10⁵ Pa et 25*10⁵ Pa.

3. Procédé selon la revendication 1 ou 2, le cis-2-butène et le trans-2-butène contenus dans le mélange de réactants, ainsi que le 1-butène également éventuellement contenu dans le mélange de réactants, formant ensemble un substrat, **caractérisé en ce que** 96% à 99% du substrat transformé est transformé en n-pentanal (régiosélectivité en ce qui concerne le n-pentanal) .

4. Procédé selon la revendication 3, réalisé dans une période de temps d'au moins 2000 h, **caractérisé en ce que** la régiosélectivité en ce qui concerne le n-pentanal se situe dans la plage de 96% à 99% pendant toute la période de temps ; mis à part des valeurs aberrantes isolées dues aux techniques de mesure.

5. Procédé selon la revendication 1 ou selon l'une quelconque des revendications 2 à 4, **caractérisé en ce que** l'hydroformylation est réalisée en présence d'une amine organique de formule (3), 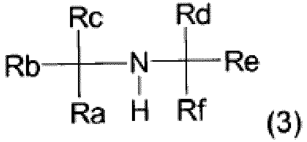 dans laquelle Ra, Rb, Rc, Rd, Re et Rf représentent des radicaux hydrocarbonés identiques ou différents, qui peuvent aussi être reliés entre eux.

6. Procédé selon la revendication 5, **caractérisé en ce que** l'amine organique présente au moins une unité 2,2,6,6-tétraméthylpipéridine.

7. Procédé selon la revendication 6, **caractérisé en ce que** l'amine organique une amine est un diester de 4-(2,2,6,6-tétraméthylpipéridinyle) de l'acide sébacique.

8. Procédé selon la revendication 1 ou selon l'une quelconque des revendications 2 à 7, **caractérisé en ce que** le mélange de réactants présente la composition suivante qui se complémente jusqu'à 100% en poids :
somme du cis-2-butène et du trans-2-butène : 10% en poids à 50% en poids ;
1-butène : 0 à 5% en poids ;
somme du n-butane et de l'isobutane : 50% en poids à 90% en poids ;
somme des autres produits : 0 à 1% en poids.

9. Procédé selon la revendication 1 ou selon l'une quelconque des revendications 2 à 8, **caractérisé en ce que** la concentration en rhodium dans le premier mélange réactionnel est de 1 ppm en poids à 1000 ppm en poids, et **en ce que** le rapport ligand/rhodium se situe entre 1:1 et 100:1, aucun ligand autre que le composé organophosphoré selon la formule (1) n'étant prévu en tant que partie du système de catalyseur homogène.
